(19) **Europäisches Patentamt / European Patent Office / Office européen des brevets**

(11) **EP 1 820 530 B1**

(12) # EUROPEAN PATENT SPECIFICATION

(45) Date of publication and mention
of the grant of the patent:
**08.10.2014 Bulletin 2014/41**

(51) Int Cl.:
***A61M 16/16*** *(2006.01)*

(21) Application number: **06110150.7**

(22) Date of filing: **20.02.2006**

(54) **Humidifier and use of the humidifier**

Befeuchter und Verwendung eines Befeuchters

Humidificateur et utilisation d'un humdificateur

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**22.08.2007 Bulletin 2007/34**

(73) Proprietor: **Thulin, Björn**
**115 37 Stockholm (SE)**

(72) Inventors:
• **Thulin, Björn**
**115 37, Stockholm (SE)**

• **Trepte, Oliver**
**192 69, Sollentuna (SE)**

(74) Representative: **Holme Patent A/S**
**Valbygårdsvej 33**
**2500 Valby (DK)**

(56) References cited:
**EP-A- 1 262 209      EP-A1- 1 671 668**
**WO-A-02/085417      US-A- 4 355 636**
**US-A- 4 367 734      US-A- 5 062 145**
**US-A- 5 738 808      US-A- 5 996 976**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of Invention

The present invention relates to a humidifier comprising a container and a semi-permeable membrane dividing the container into a water part, which in the operational state of the humidifier is filled at least partly with water, and an air part, which has an inlet and an outlet for an airflow to be humidified.

The invention also relates to a use of the humidifier for humidifying an airflow to a breathing device of the kind, which is used for persons needing artificial breathing or assisted breathing.

Background of the Invention

Artificial breathing or assisted breathing is frequently used e.g. in connection with snoring and the obstructive apnea syndrome (OSAS), and it can inter alia be performed by means such as lung ventilation, CPAP (Continuous Positive Airway Pressure). Sometimes there is however a need for humidifying the air before it leaves the apparatus used for the purpose and enters the user.

Description of the Prior Art

A known humidifier for humidifying breathing air is of the pass-over type, i.e. an apparatus in which the air is flowing over a heated water bath.

The breathing air is in another known humidifier passing through a semi-permeable hose, such as a Gore™ or a Nafion™ hose, which is surrounded by water holding a distinct temperature. The airflow and the water temperature together with the wall thickness of the hose are parameters, which are influencing the needed length and diameter of such hose.

Said prior art humidifiers function satisfactorily but suffer on the other hand from the drawback that they take up a considerable space, thereby making them impractical to use in compact devices.

From US 4,355,636 is known a humidifier and heater for air to be inhaled for connection to an inhalation conduit of a respirator. This known humidifier has a housing holding a bundle of vapour permeable fiber tubes having evaporation fiber wall surfaces. Warm water circulates in and out of the humidifier via an inlet and an outlet, respectively of the humidifier.

A humidifier for an inhalant gas is disclosed in US 5,738,808. A bundle of narrow fibers made of water-vapour permeable membrane are immersed in water so that the inhalant gas when transported through the fibers is humidified.

Summary of the Present Invention

A first aspect of the present invention is to provide a humidifier, which occupies a little space.

A second aspect of the present invention is to provide a humidifier with a filtration efficiency > 99.999% at a particle size of 0,1 μm.

A third aspect of the present invention is to provide a use of the humidifier.

The new and unique way in which the present invention fulfills the above-mentioned aspects is by providing an apparatus as mentioned in the opening paragraph wherein the semi-permeable membrane comprises a number of hoses with semi-permeable walls.

The above-mentioned known humidifier functions with a semi-permeable hose, which has a perimeter surface large enough for allowing the needed amount of breathing air molecules to pass through the semi-permeable wall of the hose. That imply that the hose and thereby the humidifier will get an impractical large size.

By arranging in parallel a number of semi-permeable hoses, this means more than two hoses, having each a relatively smaller diameter than the hoses used in conventional humidifiers, a large air/water surface can be obtained in a smaller space than in prior art humidifiers.

The ability of a humidifier based on a semi-permeable membrane to humidify passing air depends on, among other things, the water temperature, the pore size and density of the membrane, the surface of the membrane, and the airflow. When temperature, air flow, pore size and density have been given, what remains is the membrane surface. A number of hoses in parallel have a perimeter surface defined by the equation:

$$A_{peri} = 2 \cdot \pi \cdot l \cdot r \cdot n$$

where $A_{peri}$ is the area, l is the length, r is the radius of the hoses, and n is the number of hoses. The hose volume, V, is defined by the equation:

$$V = l \cdot \pi \cdot r^2 \cdot n$$

In order to keep the airflow resistance at an acceptable level, a certain minimum cross section needs to be maintained for the airflow. The total airflow cross section $A_\phi$, for the parallel hoses is defined by the equation:

$$A_\phi = \pi \cdot r^2 \cdot n$$

The equations above teach that:

- The number of parallel hoses needed to maintain the minimum airflow cross section is proportional to the inverse of the square of the hose radius.

- The perimeter surface is proportional to the hose radius, while the volume taken up is proportional to the square of the radius.

- At constant flow cross-section, and constant perimeter area, the volume taken up by a bundle of parallel hoses is inversely proportional to the number n of hoses.

Hence, it can be concluded that four hoses, each having a diameter of 1/2, compared to one single hose with a diameter of 1, produce the same air flow cross section, need half the length, and take up half the volume.

In another example sixteen parallel hoses having each a diameter of 1/4 of the diameter of the standard diameter hose produce the same flow area and thereby the same flow resistance as the standard hose. Said sixteen hoses therefore together have a surface of 16 * 1/4 = 4 times the surface of the standard diameter hose having the same length. The length of the package of small hoses thus could be reduced to 1/4 and the package would still deliver the same amount of humidity.

In a preferred embodiment the number of hoses can be made of a material which prevent passage of vira and bacteria. By e.g using a Gore™ Medical Membranes, obtainable from W. L. Gore & Associates, Inc., 555 Papermill Road, Newark, DE 19711, U.S.A, with a filtration efficiency > 99.999% at a particle size of 0,1 $\mu$m for the number of hoses, the most complete and dependable sterile barrier to bacterial and viral contamination is obtained, including:

• Best bacterial and viral control available,
• Cost effective solutions for new and existing designs,
• Qualified for use in medical devices according to USP testing,
• Suited for use with ethylene oxide (etO), steam, gamma or electron beam sterilization,
• Proven performance and reliability in hospitals and clinics since 1985.
The number of hoses can in an advantageous embodiment according to the invention, be arranged in at least two rows and the hoses in one of the rows can moreover be displaced transversely in relation to the hoses in neighbouring rows whereby the bundle of hoses will occupy a reduced space.

Brief Description of the Figures

The invention will be explained in greater detail below where further advantageous properties and example embodiments are described with reference to the drawing, where

Fig. 1 shows schematically a first embodiment of the humidifier according to the invention, seen in section from the side,

Fig. 2 is a sectional view taken along the line II - II in fig. 1,

Fig. 3 Fig. 1 shows schematically a second embodiment of the humidifier according to the invention, seen in section from the side, and

Fig 4 is a sectional view taken along the line IV - IV in fig. 3.

Description of Preferred Embodiments of the Invention

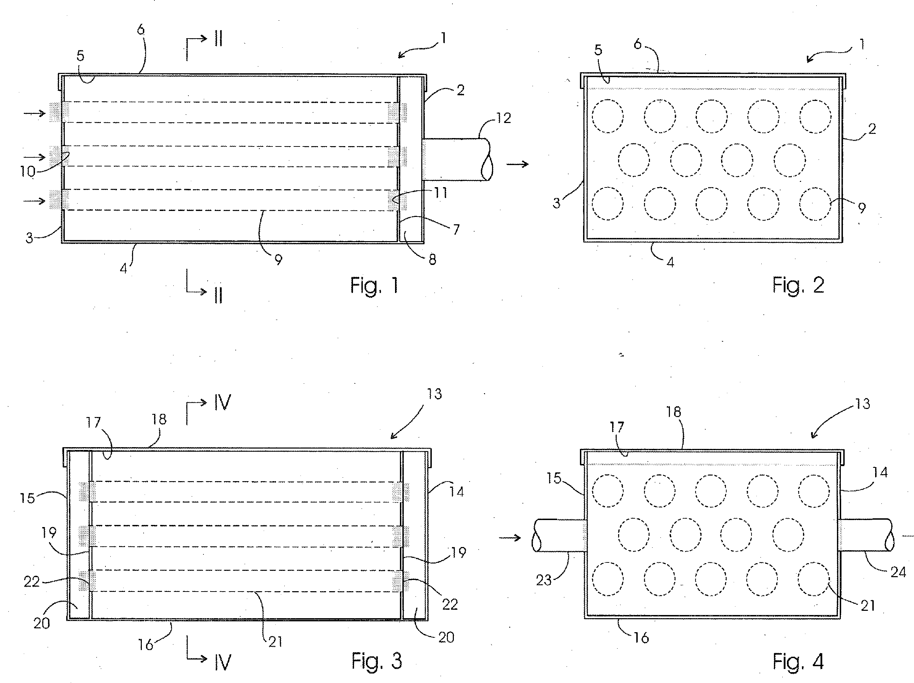

Fig. 1 and 2 shows schematically a first embodiment 1 of the humidifier according to the invention comprising a container 2, which - seen in the figures - has a vertical wall 3, a bottom 4 and a top opening 5, which allows access to the interior of the container for inspection and washing. A removable lid 6 serves for closing the opening. A partition 7 delimits a chamber 8 in the container.

In this case fourteen open hoses 9 are mounted in parallel in the container 1. Each of the hoses is extending between an opening 10 in the wall 3 and an opening 11 in partition 7. The chamber 8 thus serves as a manifold 8 for the hoses. Each hose 9 consist moreover of a semipermeable membrane, e.g. a Gore™ Medical Membrane.

The hoses are, as seen in fig. 2, staggered mounted in relation to each other whereby they advantageously obtain a compact configuration.

The humidifier 1 is advantageously used for humidifying an air flow to a breathing device (not shown) used by persons needing artificial breathing or assisted breathing, whereby the container is filled with water (not shown), which surrounds the hoses.

As seen best in fig. 2 the hoses are in this case regularly distributed over the sectional area of the container. The hoses can however, within the scope of the invention, instead be placed closer to the bottom of the container so that a space without hoses is formed above the upper hoses in the container. This space can contain a quantity of water, which can be used to humidify the air within a period. The embodiment is especially advantageous when the humidifier is used to a sleeping person.

By means of a not shown pump is air, when using the humidifier, propelled in the direction of the arrows into the manifold 8 via the openings 10 in the walls 3, the hoses 9, and the openings 11 in the partition 7 and then to the breathing device via a common hose 12 which connects the manifold and the breathing device.

The air supplied by the pump will often be relatively dry. The user therefore needs the air to be humidified. This humidifying takes place when the air is flowing through the semi-permeable hoses because air molecules then pass from the surrounding water in the container to the interior of the hoses via their semi-permeable walls.

The humidifier of the invention can advantageously be used in connection with the breathing device disclosed in the applicant's patent application WO 03/099363 A1.

Fig. 3 and 4 shows schematically a second embodiment 13 of the humidifier according to the invention comprising a container 14, which - seen in the figures - has a vertical wall 15, a bottom 16 and a top opening 17, which allows access to the interior of the container for inspection and washing. A removable lid 18 serves for closing the opening. In this case two partitions 19 are delimiting two chambers 20 in the container.

Fourteen open hoses 21 are - in this case - mounted in parallel in the container 13. Each of the hoses is extending between an opening 22 in each of the partitions 19. Each hose 21 consist of a semi-permeable membrane, e.g. a Gore™ Medical Membrane.

The hoses 21 and the chambers 20 are in the second embodiment 13 of the humidifier filled with water (not shown) while air is surrounding the hoses 21.

The humidifier 13 can advantageously be used for humidifying an airflow to a breathing device in the same way as the first embodiment 1 according to the invention.

The breathing air is however in this case, by using the humidifier 13, humidified by passing on the outside of the hoses whereby the air is supplied to the container via a common hose 23 while another common hose 24 connects the container with a breathing device (not shown).

The second embodiment of the humidifier has the advantage of easily being able to offer the airflow a sufficient large flow area and also generate a turbulent flow thereby increasing the humidifying process.

The second embodiment of the humidifier functions moreover in the same way as the first embodiment of the humidifier and a description of this function will therefore not be repeated here.

Above is described and on the drawing shown that the humidifiers have fourteen hoses. The humidifiers can however within the scope of the invention have any other appropriate numbers of hoses.

Also, the shape of the container can within the scope of the invention have any other appropriate shape than the shape of the containers shown in the drawing.

**Claims**

1. A humidifier comprising a container (2;14) that has a top opening (5;17), a vertical wall (3;15), a bottom (4;16), and a semi-permeable membrane dividing the container into a water part, which in the operational state of the humidifier is filled at least partly with water, and an air part, which has an inlet and an outlet for an airflow to be humidified, the semi-permeable membrane comprises a number of hoses (9;21) with semi-permeable walls, the container (2;14) having removable lid (6;18) adapted to cover the top opening (5;17), **characterized in that** the container further comprises a partition (7;19) that delimits a chamber (8;20) in the container (2;14), which chamber (8;20) serves as a manifold for the hoses.

2. A humidifier according to claim 1, **characterized in that** the hoses (9;21) are connected in parallel.

3. A humidifier according to claim 1 or 2, **characterized in that** the hoses (9;21) are arranged in at least two rows.

4. A humidifier according to claim 3, **characterized in that** the hoses (9;21) in one row are displaced transversely in relation to the hoses (9;21) in neighbouring rows.

5. A humidifier according to any of the claims 1 - 4, **characterized in that** the water part is outside the hoses (9) while the air part is inside the hoses (9).

6. A humidifier according to claim 5, **characterized in that** the humidifier comprises the manifold (8) at least at the outlet of the hoses (9).

7. A humidifier according to any of the claims 1 - 4, **characterized in that** the water part is inside the hoses (21) while the air part is outside the hoses (21).

8. A humidifier according to claim 7, **characterized in that** the humidifier comprises manifolds (20) at both ends of the hoses (21).

9. A humidifier according to any of the claims 1 - 8, **characterized in that** the hoses (9;21) is made of a material which prevent passage of vira and bacteria.

10. A humidifier according to claim 9, **characterized in that** the material which prevent passage of vira and bacteria has a filtration efficiency >99.999% at a particle size of 0,1 $\mu$m for the number of hoses.

11. Use of the humidifier according to claim 1 - 10 for humidifying an air flow to a breathing device of the kind which is used for persons needing artificial breathing or assisted breathing.

**Patentansprüche**

1. Luftbefeuchter mit einem Behälter (2; 14), der eine obere Öffnung (5; 17), eine vertikale Wand (3; 15), einen Boden (4; 16) und eine semipermeable Membran aufweist, die den Behälter in einen Wasserabschnitt, der im Betriebszustand des Luftbefeuchters wenigstens teilweise mit Wasser gefüllt ist, und einen Luftabschnitt, der einen Einlass und einen Auslass für einen zu befeuchtenden Luftstrom aufweist, unterteilt, wobei die semipermeable Membran eine Anzahl von Schläuchen (9; 21) mit semipermeablen Wandungen aufweist, und der Behälter (2; 14) einen entfernbaren Deckel (6; 18) hat, der zum Abdecken der oberen Öffnung (5; 17) eingerichtet ist, **dadurch gekennzeichnet, dass** der Behälter weiter eine Unterteilung (7; 19) aufweist, die eine Kammer (8; 20) im Behälter (2; 14) begrenzt, wobei die Kammer (8; 20) als Verteiler für die Schläuche dient.

2. Luftbefeuchter nach Anspruch 1, **dadurch gekennzeichnet, dass** die Schläuche (9; 21) parallel angeschlossen sind.

3. Luftbefeuchter nach Anspruch 1 oder 2, **dadurch gekennzeichnet, dass** die Schläuche (9, 21) in wenigstens zwei Reihen angeordnet sind.

4. Luftbefeuchter nach Anspruch 3, **dadurch gekennzeichnet, dass** die Schläuche (9; 21) in einer Reihe in Relation zu Schläuchen (9; 21) in benachbarten Reihen schräg versetzt sind.

5. Luftbefeuchter nach einem der Ansprüche 1 bis 4, **dadurch gekennzeichnet, dass** der Wasserabschnitt außerhalb der Schläuche (9) ist, wohingegen der Luftabschnitt innerhalb der Schläuche (9) ist.

6. Luftbefeuchter nach Anspruch 5, **dadurch gekennzeichnet, dass** der Luftbefeuchter den Verteiler (8) wenigstens am Auslass der Schläuche (9) aufweist.

7. Luftbefeuchter nach einem der Ansprüche 1 bis 4, **dadurch gekennzeichnet, dass** der Wasserabschnitt innerhalb der Schläuche (21) ist, wohingegen der Luftabschnitt außerhalb der Schläuche (21) ist.

8. Luftbefeuchter nach Anspruch 7, **dadurch gekennzeichnet, dass** der Luftbefeuchter Verteiler (20) an beiden Enden der Schläuche (21) aufweist.

9. Luftbefeuchter nach einem der Ansprüche 1 bis 8, **dadurch gekennzeichnet, dass** die Schläuche (9; 21) aus einem Material gefertigt sind, das das Hindurchtreten von Viren und Bakterien verhindert.

10. Luftbefeuchter nach Anspruch 9, **dadurch gekennzeichnet, dass** das Material, das das Hindurchtreten von Viren und Bakterien verhindert, einen Filtrationswirkungsgrad von >99,999 % bei einer Partikelgröße von 0,1 $\mu$m für die Anzahl von Schläuchen hat.

11. Verwendung des Luftbefeuchters nach den Ansprüchen 1 bis 10 zum Befeuchten eines Luftstroms für ein Beatmungsgerät der Art, die für Personen verwendet wird, die künstliche Beatmung oder unterstützende Beatmung benötigen.

## Revendications

1. Humidificateur comprenant un récipient (2 ; 14) qui possède une ouverture supérieure (5 ; 17), une paroi verticale (3 ; 15), une base (4 ; 16) et une membrane semi-perméable qui subdivise le récipient en une partie pour l'eau qui, à l'état opérationnel de l'humidificateur, est remplie au moins en partie avec de l'eau, et une partie pour l'air qui possède une entrée et une sortie pour un courant d'air qui doit être humidifié, la membrane semi-perméable comprenant plusieurs tuyaux flexibles (9 ; 21) comportant des parois semi-perméables, le récipient (2 ; 14) possédant un couvercle amovible (6 ; 18) conçu pour fermer l'ouverture supérieure (5 ; 17), **caractérisé en ce que** le récipient comprend en outre une séparation (7 ; 19) qui délimite une chambre (8 ; 20) dans le récipient (2 ; 14), ladite chambre (8 ; 20) servant de collecteur pour les tuyaux flexibles.

2. Humidificateur selon la revendication 1, **caractérisé en ce que** les tuyaux flexibles (9 ; 21) sont montés en parallèle.

3. Humidificateur selon la revendication 1 ou 2, **caractérisé en ce que** les tuyaux flexibles (9 ; 21) sont disposés en au moins deux rangées.

4. Humidificateur selon la revendication 3, **caractérisé en ce que** les tuyaux flexibles (9 ; 21) dans une rangée sont décalés en direction transversale par rapport aux tuyaux flexibles (9 ; 21) dans les rangées voisines.

5. Humidificateur selon l'une quelconque des revendications 1 à 4, **caractérisé en ce que** la partie pour l'eau est située à l'extérieur des tuyaux flexibles (9), tandis que la partie pour l'air est disposée à l'intérieur des tuyaux flexibles (9).

6. Humidificateur selon la revendication 5, **caractérisé en ce que** l'humidificateur comprend le collecteur (8) au moins à la sortie des tuyaux flexibles (9).

7. Humidificateur selon l'une quelconque des revendications 1 à 4, **caractérisé en ce que** la partie pour l'eau est située à l'intérieur des tuyaux flexibles (21), tandis que la partie pour l'air est disposée à l'extérieur des tuyaux flexibles (21).

8. Humidificateur selon la revendication 7, **caractérisé en ce que** l'humidificateur comprend des collecteurs (20) aux deux extrémités des tuyaux flexibles (21).

**9.** Humidificateur selon l'une quelconque des revendications 1 à 8, **caractérisé en ce que** les tuyaux flexibles (9 ; 21) sont constitués d'un matériau qui empêche le passage des virus et des bactéries.

**10.** Humidificateur selon la revendication 9, **caractérisé en ce que** le matériau qui empêche le passage des virus et des bactéries possède une efficacité de filtration > 99,999 % à une granulométrie de 0,1 $\mu$m pour le nombre de tuyaux flexibles.

**11.** Utilisation de l'humidificateur selon les revendications 1 à 10 pour humidifier un courant d'air dirigé vers un dispositif de respiration du type qui est utilisé pour des personnes ayant besoin d'une respiration artificielle ou d'une respiration assistée.

Fig. 2

Fig. 4

Fig. 1

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4355636 A
- US 5738808 A

- WO 03099363 A1